# EUROPÄISCHE PATENTSCHRIFT

(11) **EP 2 656 769 B1**
(45) Veröffentlichungstag und Bekanntmachung des Hinweises auf die Patenterteilung: **08.06.2016**
(21) Anmeldenummer: 12401074.5
(22) Anmeldetag: 27.04.2012
(51) Int. Cl.: A61B 90/70

(54) **Reinigungs- und Desinfektionsautomat**
Cleaning and disinfection machine
Automate de nettoyage et de désinfection

(43) Veröffentlichungstag der Anmeldung: 30.10.2013
(73) Patentinhaber: Miele & Cie. KG, 33332 Gütersloh (DE)
(72) Erfinder: Heitmann, Michael, 33739 Bielefeld (DE)

(56) Entgegenhaltungen:
- DE-A1- 2 919 140
- US-A- 3 969 137

## Beschreibung

Die Erfindung betrifft einen Reinigungs- und Desinfektionsautomat insbesondere für medizinische Instrumente und Geräte, mit einem einen Spülraum bereitstellenden Spülbehälter und einer im Spülraum angeordneten Sprüheinrichtung zur Beschickung von Spülgut mit Spülflotte, wobei die Sprüheinrichtung zur Versorgung mit Spülflotte an ein Versorgungsrohr angeschlossen ist.

Reinigungs- und Desinfektionsautomaten der eingangs genannten Art sind aus dem Stand der Technik an sich gut bekannt. Sie verfügen über einen Spülbehälter, der einen Spülraum bereitstellt. Im bestimmungsgemäßen Verwendungsfall dient der Spülraum der Aufnahme von zu reinigendem und/oder zu desinfizierendem Spülgut, bei dem es sich beispielsweise um medizinische Instrumente und Geräte handeln kann. Der Spülraum ist über eine Beschickungsöffnung des Spülbehälters zugänglich, die mittels einer verschwenkbar am Spülbehälter angeordneten Tür fluiddicht verschließbar ist.

Typischerweise kommt im bestimmungsgemäßen Verwendungsfall zur Spülgutreinigung ein Spülkorb zum Einsatz. Dieser wird mit dem zu reinigenden Spülgut bestückt und anschließend verwenderseitig durch die Beschickungsöffnung hindurch in den Spülraum eingeführt. Nach erfolgreich durchgeführter Spülung, d.h. Reinigung und/oder Desinfektion des Spülguts kann der Spülkorb zusammen mit den davon aufgenommenen Spülgütern verwenderseitig dem Spülraum durch die Beschickungsöffnung wieder entnommen werden.

Innerhalb des Spülraums ist eine Sprüheinrichtung angeordnet, diese dient der Beschickung von Spülgut mit Spülflotte. Typischerweise weist die Sprüheinrichtung Sprüharme auf, die verdrehbar gelagert sind. Es kann dabei zwischen automatenseitigen Sprüharmen einerseits und korbseitigen Sprüharmen andererseits unterschieden werden. Dabei betreffen "automatenseitige Sprüharme" solche Sprüharme, die im Spülraum des Automaten installiert sind. In der Regel sind zwei solcher Sprüharme vorgesehen, wobei der eine deckenseitig und der andere bodenseitig des Spülraums ausgebildet ist. "Korbseitige Sprüharme" betreffen indes solche Sprüharme, die am Spülkorb angeordnet sind, die also zusammen mit dem Sprühkorb verwenderseitig verfahren werden können. Zum Anschluss dieser Sprüharme an ein Wasser- und/oder Spülflottenzuführsystem sind im Spülraum entsprechend ausgebildete Ankopplungsstellen vorgesehen, wie beispielsweise aus der DE 10 2007 003 894 A1 bekannt.

Die Sprüheinrichtung kann je nach Ausgestaltung des Reinigungs- und Desinfektionsautomaten nicht nur der Abgabe von Spülflotte dienen. Es sind Ausgestaltungsformen bekannt geworden, wonach die Sprüheinrichtung auch dazu dient, das Spülgut nach erfolgter Reinigung und/oder Desinfektion zu Trocknen, zu welchem Zweck Trocknungsluft in den Spülraum über die Sprüheinrichtung eingeleitet wird. In diesem Fall dient die Sprüheinrichtung einerseits dazu, während des eigentlichen Reinigungs- und/oder Desinfektionsvorganges Spülflotte auf das zu reinigende und/oder zu desinfizierende Spülgut abzugeben sowie andererseits dazu, nach Abschluss eines solchen Reinigungs- und/oder Desinfektionsvorganges das Spülgut mit Trocknungsluft zu beaufschlagen.

Um über die Sprüheinrichtung die Abgabe einerseits von Spülflotte und andererseits von Trocknungsluft zu gestatten, kommen bei Reinigungs- und Desinfektionsautomaten kleinerer Bauart entsprechende Trenn- und Verteileinrichtungen zum Einsatz. Diese sorgen für eine Beschickung der Sprüheinrichtung entweder mit Spülflotte oder mit Trocknungsluft.

Aus dem Stand der Technik sind auch Konstruktionen bekannt geworden, die nicht über Trenn- und Verteileinrichtungen der vorbeschriebenen Art verfügen. Bei solchen Automaten handelt es sich typischerweise um Großgeräte, die über eine Mehrzahl von Sprüharmen oder dergleichen Fluidabgabeeinrichtungen verfügen, die typischerweise in Höhenrichtung übereinander angeordnet sind. Zur Versorgung dieser Sprüharme entweder mit Spülflotte oder mit Trocknungsluft sind jeweils Speiseleitungen vorgesehen, die über außerhalb des Spülraums ausgebildete Verteileinrichtungen strömungstechnisch miteinander koppelbar bzw. voneinander entkoppelbar sind. Ein solcher Aufbau ist vergleichsweise kompliziert und störanfällig und hat aus diesem Grunde bislang nur bei Großgeräten Einsatz gefunden.

Sowohl bei Reinigungs- und Desinfektionsautomaten kleinerer Bauart als auch bei Großgeräten kommt zum Zwecke der Umwälzung von Spülflotte eine Umwälzpumpe zum Einsatz. Über diese wird im Spülraum befindliche Spülflotte angesaugt und über entsprechende Versorgungsrohre oder -leitungen Sprüharmen oder sonstigen Fluidabgabeeinrichtungen der Sprüheinrichtung zugeführt. Derlei Versorgungsrohre sind zur Maximierung der Größe des nutzbaren Spülraums außerhalb des Spülraums verlegt, wie sich dies beispielsweise aus der DE 10 2009 009 768 A1 und der DE 10 2006 009 787 A1 ergibt. Die Verlegung der Versorgungsrohre außerhalb des Spülraums ist darüber hinaus mit Blick auf die schon vorbeschriebene Ankopplung von korbseitigen Sprüharmen an ein Fluidzuführungssystem von Vorteil.

Die DE 2919149 A1 beschreibt darüber hinaus eine Spülmaschine mit einem zentral im Spülraum installierten Versorgungsrohr zur Versorgung von Sprüheinrichtungen mit Spülflotte, welches mit der Spülwasserversorgung verbunden ist.

Es ist deshalb ausgehend von dem Vorbeschriebenen die Aufgabe der Erfindung, einen neuartigen Reinigungs- und Desinfektionsautomaten vorzuschlagen, der bei gleichzeitiger Verbesserung der Betriebssicherheit eine vereinfachte Handhabung gestattet.

Zur Lösung dieser Aufgabe wird mit der Erfindung ein Reinigungs- und Desinfektionsautomat mit den Merkmalen von Anspruch 1 vorgeschlagen.

Nach dem aus dem Stand der Technik bekannten, bisherigen Konstruktionsprinzip erfolgt eine Anbindung der Sprüheinrichtung an die Umwälzpumpe über Versorgungsrohre, die außerhalb des Sprühraums ausgebildet sind. Die Anordnung der Versorgungsrohre außerhalb des Spülraums ist insbesondere deshalb vorgesehen, um die nutzbare Größe des Spülraums zu maximieren.

Mit der Erfindung wird nun ein völlig neues Konstruktionsprinzip vorgeschlagen und insofern ein völlig anderer Lösungsweg beschritten. Nach der erfindungsgemäßen Ausgestaltung ist vorgesehen, in Abkehr zum bisherigen Konstruktionsprinzip das zur Versorgung der Sprüheinrichtung mit Spülflotte vorgesehene Versorgungsrohr innerhalb des Spülraums anzuordnen. Außerhalb des Spülraums vorgesehene Versorgungsrohre entfallen demnach vollständig.

Die erfindungsgemäße Ausgestaltung tritt dem in der Fachwelt vorherrschenden Vorurteil entgegen, die Anordnung eines Versorgungsrohres im Spülraum würde die nutzbare Größe des Spülraums verkleinern. Das Gegenteil ist tatsächlich der Fall. Da auf außerhalb des Spülraums verlegte Versorgungsrohre vollständig verzichtet ist, kann der hierdurch frei werdende Bauraum für eine Spülraumvergrößerung genutzt werden, und dies bei gleichbleibenden Außenabmessungen des Reinigungs- und Desinfektionsautomatens. Im Ergebnis bringt die neue Konstruktion mithin einen vergrößerten Spülraum, der den für das Versorgungsrohr benötigten Platzbedarf innerhalb des Spülraums mehr als aufwiegt. Es kann damit insgesamt eine Kapazitätserweiterung erreicht werden, was dazu beiträgt, Ressourcen schonen zu können.

Das nach der Erfindung vorgesehene Versorgungsrohr durchragt den Spülraum. Einzelne Sprüharme und/oder sonstige Fluidabgabeeinrichtungen der Sprüheinrichtung sind zur Versorgung mit Spülflotte an das Versorgungsrohr angeschlossen. Es erfolgt insofern eine direkte Beschickung der Sprüharme und/oder sonstigen Fluidabgabeeinrichtungen mit Spülflotte über das Versorgungsrohr. Da das Versorgungsrohr innerhalb des Spülraums angeordnet ist, sinkt im Unterschied zum Stand der Technik das Risiko von Leckagen auf ein Minimum. Darüber hinaus werden zum Stand der Technik die Wasser- bzw. Spülflottenwege erheblich verkürzt, womit einerseits eine vereinfachte Restwasserentleerung stattfinden kann und andererseits Bypass-Spülungen zur Selbstdesinfektion aufgrund des Wegfalls verzweigter Wasser- bzw. Spülflottenwege erheblich minimiert werden können.

Darüber hinaus erweist sich die erfindungsgemäße Ausgestaltung insofern als Vorteil, als dass im Unterschied zum Stand der Technik auf verzweigte Wasserwege und Umlenkungen außerhalb des Spülraums verzichtet ist, womit im bestimmungsgemäßen Verwendungsfall auftretende Strömungsverluste minimiert werden. Auch insofern erweist sich die erfindungsgemäße Konstruktion als Ressourcen schonend.

Das Versorgungsrohr ist gemäß einem weiteren Merkmal der Erfindung einendseitig an eine Umwälzpumpe angeschlossen. Über diese wird im Betriebsfall umgewälzte Spülflotte direkt in das Versorgungsrohr eingeleitet.

In diesem Zusammenhang ist besonders bevorzugt, dass der Sammeltopf, in den der Spülbehälter mündet, der Umwälzpumpe als Pumpengehäuse dient, d.h. der Sammeltopf und das Pumpengehäuse als ein Bauteil ausgebildet sind. Vorzugsweise ist die Umwälzpumpe im Vergleich zu den aus dem Stand der Technik bekannten Konstruktionen um 90° verdreht ausgerichtet, d.h. das Pumpenrad der Umwälzpumpe und die im Spülraum angeordneten Sprüharme der Sprüheinrichtung drehen um ein und dieselbe Drehachse. Hierdurch ist ein besonders strömungsoptimierter Wasserweg realisiert. Bei aus dem Stand der Technik bekannten Konstruktionen sind die Drehachse des Pumpenrades einerseits und die Drehachse der Sprüharme andererseits quer zueinander ausgerichtet, in der Regel unter Ausbildung eines 90°-Winkels.

Das Pumpenrad der Umwälzpumpe wirkt mit einem fest- bzw. stillstehenden Leitrad zusammen. Die im Betriebsfall am Umfang des Pumpenrades austretende Wassermenge wird durch dieses Leitrad aufgefangen und zur Drehachse hin umgelenkt, so dass das Wasser ohne weitere Umwege und Umlenkungen in Richtung der Drehachse in das im Innenraum des Automaten angeordnete Versorgungsrohr eingeleitet werden kann.

An der tiefsten Stelle des Sammeltopfes bzw. des Pumpengehäuses ist bevorzugter Weise ein Ablaufstutzen vorgesehen. Über diesen kann eine Spülflotten- bzw. Restwasserentleerung stattfinden, was im Unterschied zum Stand der Technik mangels aufwändiger Verzweigungswege sehr viel einfacher und besser erfolgen kann. In diesem Zusammenhang ist es ferner bevorzugt, das den Sammeltopf bzw. das Pumpengehäuse gegenüber dem weiteren Spülraum begrenzende Flächensieb mit einem Reservoir direkt vor dem Ablaufstutzen auszurüsten, in welchem sich während eines bestimmungsgemäßen Spülprogramms gröbere Partikel, Verschmutzungen und/oder dergleichen ansammeln können, die nicht durch das Flächensieb hindurchgeführt werden können, so dass sie bei einem Spülflottenablauf über den Ablaufstutzen mit weggespült werden.

Das Versorgungsrohr ist gemäß einem weiteren Merkmal der Erfindung anderendseitig an ein Gebläse angeschlossen. Die Sprüheinrichtung kann in diesem Fall auch zur Beaufschlagung des Spülguts mit Trocknungsluft genutzt werden. Die vom Gebläse erzeugte Trocknungsluft wird in das Versorgungsrohr eingeleitet, von wo aus die einzelnen Sprüharme und/oder sonstigen Fluidabgabeeinrichtungen der Sprüheinrichtung mit Trocknungsluft versorgt werden.

Um im Trocknungsfall eine strömungstechnische Trennung von Umwälzpumpe und Versorgungsrohr zu gestatten, ist gemäß einem weiteren Merkmal der Erfindung eine zwischen Umwälzpumpe einerseits und Versorgungsrohr andererseits ausgebildete Ventileinrichtung vorgesehen. Diese ist im Fall des Betriebs der Umwälzpumpe geöffnet. Andernfalls ist sie geschlossen, insbesondere wenn über das Gebläse Trocknungsluft in das Versorgungsrohr eingeleitet wird.

Ein besonders kompakter Aufbau wird gemäß einem weiteren Merkmal der Erfindung erreicht, wenn die Mittelachse des Sprühraums und die Drehachse der Sprüharme zusammen fallen. Dabei ist der Spülraum in seinen Grundabmessungen bevorzugter Weise quadratisch ausgebildet. Da die Mittelachse des Sprühraums und die Drehachse der Sprüharme zusammen fallen, können in ihren geometrischen Abmessungen Sprüharme gewählt werden, die eine optimierte Beschickung des Spülraums mit Spülflotte bewirken. Insbesondere die von der Mittelachse entfernten Eckbereiche des Spülraums können bei einer derartigen Ausgestaltung noch mit erfasst und deshalb mit Spülflotte versorgt werden. Die aus dem Stand der Technik bekannten Konstruktionen verfügen im Unterschied hierzu über einen in der Grundfläche rechteckförmig ausgestalteten Spülraum, was es erforderlich macht, die Sprüharmgeometrie auf die kürzeste Seite des rechteckförmigen Spülraums auszurichten. In der Konsequenz können insbesondere die Eckbereiche bei aus dem Stand der Technik bekannten Einrichtungen nicht in hinreichendem Maß sicher mit Spülflotte versorgt werden. Die erfindungsgemäße Konstruktion schafft hier aus den schon vorerläuterten Gründen in vorteilhafter Weise Abhilfe.

Das Versorgungsrohr besteht gemäß einem weiteren Merkmal der Erfindung aus einzelnen Abschnitten, die fluiddicht miteinander verbindbar sind. Dabei ist es bevorzugt, dass das Versorgungsrohr einen ersten und einen zweiten Abschnitt aufweist, die automatenseitig ausgebildet sind. Ein weiterer Abschnitt des Versorgungsrohrs ist indes korbseitig ausgebildet und ist zusammen mit dem Spülkorb dem Spülraum entnehmbar bzw. in diesen hineinführbar.

Bei den automatenseitigen Abschnitten des Versorgungsrohres handelt es sich bevorzugter Weise um zwei Abschnitte, die bodenseitig und deckenseitig des Spülraums montiert sind. Ein jeder dieser beiden Abschnitte ist mit einem entsprechenden Sprüharm gekoppelt. Im bestimmungsgemäßen Verwendungsfall ist der weitere Abschnitt des Versorgungsrohrs fluiddicht mit den beiden automatenseitigen Abschnitten verbunden, so dass über die Umwälzpumpe geförderte Spülflotte einerseits den bodenseitigen Sprüharm und über das Versorgungsrohr auch den deckenseitigen Sprüharm erreicht. Zur Kupplung der automatenseitigen Abschnitte des Versorgungsrohrs mit dem weiteren, korbseitigen Abschnitt des Versorgungsrohrs dienen jeweils Kupplungsstellen, die für eine fluiddichte Verbindung der einzelnen Abschnitte sorgen. Dabei kann der korbseitige Abschnitt des Versorgungsrohrs seinerseits in einzelne Unterabschnitte oder Segmente unterteilt sein, was sich insbesondere dann anbietet, wenn der Spülkorb aus einzelnen Unterkörben zusammengesetzt ist. In diesem Fall ist jeder Unterkorb mit einem Versorgungsrohrsegment ausgerüstet. Die Unterkörbe werden zu einem Spülkorb zusammengestellt, wobei die einzelnen Segmente des Versorgungsrohrs fluiddicht miteinander zum mittleren Abschnitt des Versorgungsrohrs miteinander gekoppelt werden. Dieser mittlere Abschnitt wird dann im bestimmungsgemäßen Anwendungsfall zusammen mit dem Spülkorb in den Spülraum des Automatens eingebracht und dort fluiddicht mit den automatenseitigen Abschnitten des Versorgungsrohrs gekoppelt, so dass insgesamt eine fluiddichte Ausgestaltung vorliegt.

Der korbseitige Abschnitt des Versorgungsrohrs kann, ob nun in einzelne Segmente unterteilt oder nicht, seinerseits an weitere Sprüharme angeschlossen sein. Bei diesen Sprüharmen handelt es sich dann um korbseitige Sprüharme.

Die koaxiale Anordnung des Versorgungsrohres zur Sprüharmdrehachse und die mechanische, fluiddichte Verbindung aller Sprüharme über das Versorgungsrohr ermöglicht eine besonders einfache Übertragung des Antriebsmomentes in die Sprüheinrichtung, insbesondere in die an den korbseitigen Abschnitt des Versorgungsrohres angeschlossenen Sprüharme. Vorzugsweise sind somit ein oder mehrere korbseitigen Sprüharme über einen, insbesondere elektromotorischen Antrieb von außen antreibbar, besonders bevorzugterweise werden alle Sprüharme der Sprüheinrichtung von dem Antrieb angetrieben.

Die erfindungsgemäße Konstruktion ergibt gegenüber dem Stand der Technik insbesondere folgende Vorteile:
- Die Länge der Wasserwege und die Anzahl der Umlenkungen ist deutlich reduziert, womit eine Minimierung der Strömungsverluste erreicht ist. Gemäß der vorgeschlagenen Konstruktion fördert die Umwälzpumpe direkt in Richtung der zentralen Spülflottenführung in den Spülraum hinein.
- Gegenüber dem Stand der Technik ist eine deutliche Reduzierung des Volumens für die Wasserführung erreicht, womit die in den Führungswegen gebundene Wassermenge mit dem Vorteil reduziert ist, dass bei bestimmungsgemäßer Verwendung eine gegenüber dem Stand der Technik verringerte Menge an Frischwasser erforderlich ist.
- Die Anzahl möglicher Leckagestellen ist deutlich verringert, was die Betriebssicherheit insgesamt erhöht.
- Die Anzahl der eingesetzten Bauteile und Baukomponenten ist deutlich reduziert, was nicht zuletzt eine Kostenreduktion in der Herstellung und Wartung erbringt.
- Auf außerhalb des Spülraums verlegte Versorgungsrohre oder -leitungen wird vollständig verzichtet. Dies gestattet es, den hierdurch frei werdenden Bauraum für eine Spülraumvergrößerung zu nutzen und dies beim Vergleich zum Stand der Technik gleichbleibenden Außenabmessungen des Automatens. Im Ergebnis erbringt dies eine Kapazitätserweiterung, was ebenfalls dazu beiträgt, Ressourcen schonen zu können.
- Das Spülraumunterteil ist im Unterschied zum Stand der Technik fertigungstechnisch deutlich vereinfacht, da es als Prägeteil und nicht mehr als Tiefziehteil mit angebrachtem Schweißteil ausgebildet werden kann, was die Herstellung insgesamt vereinfacht und kostengünstiger gestaltet.
- Der Restwasser- bzw. Spülflottenablauf ist erheblich verbessert, da auf verzweigte, externe Wasserwege konstruktiv verzichtet ist, eine Entleerung solcher Wasserwege im Unterschied zum Stand der Technik also nicht weiter erforderlich ist.
- Es kann ferner auf Bypass-Spülungen für entlegene Ablaufelemente und - schläuche verzichtet werden, da derartige entlegene Ablaufelemente und - schläuche bei der erfindungsgemäßen Konstruktion nicht mehr vorhanden sind.

Es ergibt sich damit alles in allem eine gegenüber dem Stand der Technik deutlich vereinfachte Handhabung.

Weitere Vorteile und Merkmale der Erfindung ergeben sich aus der nachfolgenden Beschreibung anhand der Figuren. Dabei zeigen
Fig.1 in einer schematischen Ansicht einen Reinigungs- und Desinfektionsautomaten nach der Erfindung und
Fig. 2 in einer schematischen Darstellung insbesondere den Spülbehälter eines erfindungsgemäßen Reinigungs- und Desinfektionsautomaten.

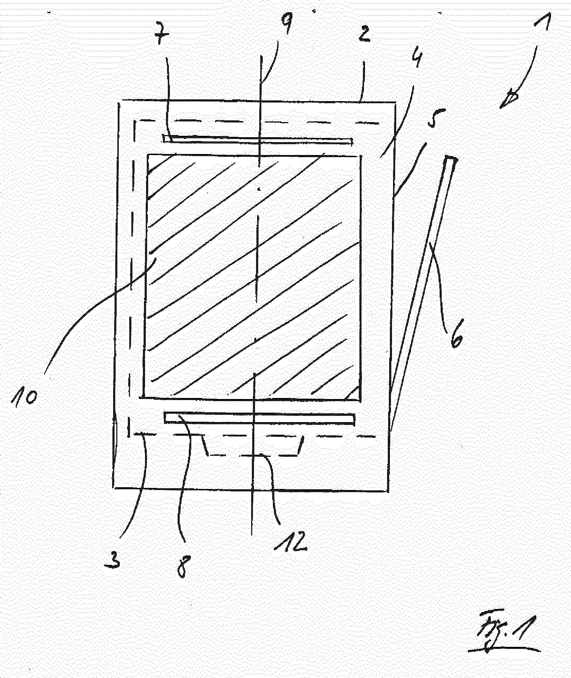

Fig. 1 lässt in schematischer Seitenansicht einen Reinigungs- und Desinfektionsautomaten nach der Erfindung erkennen, im nachfolgenden Spülautomat 1 genannt.

Der Spülautomat 1 nach der Erfindung ist als Gewerbeautomat ausgelegt und dient insbesondere der Behandlung von medizinischen Instrumenten und Geräten, beispielsweise Glaskoben, Reagenzgläsern, Schläuchen und/oder dergleichen.

Der Spülautomat 1 verfügt über ein von einem Gestell 11 getragenes Gehäuse 2. Innerhalb des Gehäuses 2 ist ein Spülbehälter 3 angeordnet, der einen Spülraum 4 bereit stellt. Der Spülraum 4 ist über eine Beschickungsöffnung 5 zugänglich, welche Beschickungsöffnung 5 mittels einer Spülraumtür 6 fluiddicht verschließbar ist.

Im bestimmungsgemäßen Verwendungsfall dient der Spülraum 4 der Aufnahme von zu reinigendem Spülgut, beispielsweise medizinischen Instrumenten und Geräten. Zur

Positionierung des zu reinigenden Spülgutes innerhalb des Spülraums 4 dient ein Spülkorb 10. Dieser kann verwenderseitig aus dem Spülraum 10 heraus verfahren bzw. in diesen eingebracht werden.

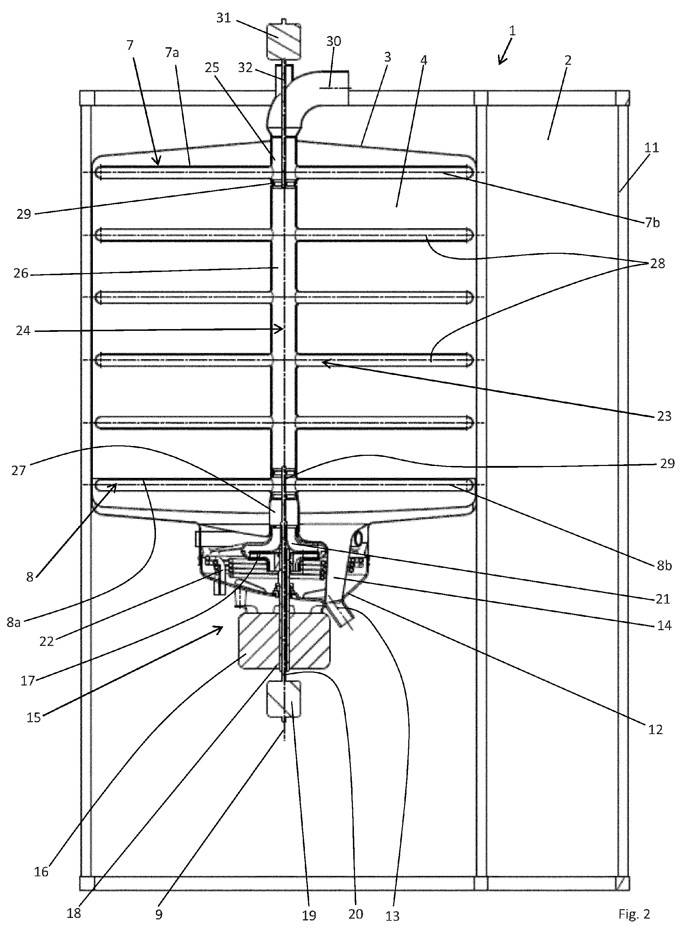

Zum Zwecke der Beschickung des Spülraums 4 mit Spülflotte oder mit Trocknungsluft kommt eine Sprüheinrichtung 23 zum Einsatz, wie sie sich insbesondere aus der Darstellung nach Fig. 2 ergibt. Die Sprüheinrichtung 23 verfügt einerseits über automatenseitige Sprüharme 7 und 8 sowie andererseits über korbseitige Sprüharme 28. Der automatenseitige Sprüharm 7 ist innerhalb des Sprühraums 4 deckenseitig montiert und verfügt über zwei Sprüharmäste 7a und 7b. Der zweite automatenseitige Sprüharm 8 ist in gleicher Weise ausgebildet und verfügt über Sprüharmäste 8a und 8b. Dabei ist der Sprüharm 8 bodenseitig des Spülraums 4 ausgebildet. Beide Sprüharme 7 und 8 drehen um die gemeinsame Drehachse 9.

Fig. 2 lässt den Aufbau des erfindungsgemäßen Spülautomatens 1 genauer erkennen. Wie dieser Darstellung zu entnehmen ist, ist der Spülbehälter 3 innerhalb des von dem Gestell 11 getragenen Gehäuses 2 angeordnet. Der Spülbehälter 3 geht bodenseitig in den Sammeltopf 12 über.

Zum Zwecke der Umwälzung der im bestimmungsgemäßen Verwendungsfall im Spülraum 4 befindlichen Spülflotte dient eine Umwälzpumpe 15. Diese saugt die sich im Sammeltopf 12 ansammelnde Spülflotte an und fördert diese zur Sprüheinrichtung 23, von wo aus dann eine Beschickung von Spülgut mit Spülflotte erfolgt.

Zur Versorgung der Sprüheinrichtung 23 mit Spülflotte dient ein Versorgungsrohr 24. Dieses Versorgungsrohr 24 ist einendseitig an die Umwälzpumpe 15 und anderendseitig an einen Luftanschluss 30 eines in den Figuren nicht näher dargestellten Gebläses angeschlossen.

Das Versorgungsrohr 24 ist erfindungsgemäß innerhalb des Spülraums 4 angeordnet. Es erstreckt sich entlang der Drehachse 9 der Sprüharme 7, 8 und 28, welche Drehachse bevorzugter Weise mit der Mittelachse des Spülbehälters 3 zusammen fällt.

Das Versorgungsrohr 24 verfügt über insgesamt drei Abschnitte 25, 26 und 27. Dabei dient der erste Abschnitt 25 als oberer Abschnitt, der mit dem deckenseitigen Sprüharm 7 gekoppelt ist. Der zweite Abschnitt 27 dient als unterer Abschnitt, der seinerseits mit dem bodenseitigen Sprüharm 8 gekoppelt ist. Der dritte Abschnitt 26 dient als mittlerer Abschnitt und ist korbseitig, d.h. als Bestandteil des dem Spülraum 4 entnehmbaren Spülkorbs 10 ausgebildet. Die Abschnitte 25, 26 und 27 sind in bestimmungsgemäßem Verwendungsfall fluiddicht miteinander verbunden.

Im einfachsten Ausführungsfall verfügt die Sprüheinrichtung 23 über die beiden automatenseitigen Sprüharme 7 und 8. Im bestimmungsgemäßen Verwendungsfall ist der dritte, d.h. der mittlere Abschnitt 26 des Versorgungsrohrs 24 fluiddicht mit den beiden anderen Abschnitten 25 und 27 verbunden, so dass über die Umwälzpumpe 15 geförderte Spülflotte einerseits den bodenseitigen Sprüharm 8 und über das Versorgungsrohr 24 auch den deckenseitigen Sprüharm 7 erreicht.

Gemäß der in den Figuren gezeigten Ausführungsform sind weitere Sprüharme 28 vorgesehen, die als spülkorbseitige Sprüharme 28 direkt an das Versorgungsrohr 24 angeschlossen sind. Im bestimmungsgemäßen Verwendungsfall findet damit über das Versorgungsrohr 24 nicht nur eine Beschickung der Sprüharme 7 und 8, sondern auch eine Beschickung der mit dem Versorgungsrohr 24 gekoppelten Sprüharme 28 statt.

Zur Kupplung des ersten Abschnitts 25 und des dritten Abschnitts 26 des Versorgungsrohrs 24 bzw. des zweiten Abschnitts 27 und des dritten Abschnitts 26 des Versorgungsrohrs 24 sind jeweils Kupplungsstellen 29 zwischen zweitem Abschnitt 27 und drittem Abschnitt 26 bzw. erstem Abschnitt 25 und drittem Abschnitt 26 vorgesehen.

Nach einer bevorzugten Ausführungsform gemäß der Darstellung nach Fig. 2 ist vorgesehen, dass der Sammeltopf 12 der Umwälzpumpe 15 als Pumpengehäuse dient, zu welchem Zweck der Sammeltopf 15 und das Pumpengehäuse als ein Bauteil ausgebildet sind. Fig. 2 lässt diese Ausgestaltung gut erkennen.

Der vom Sammeltopf 12 bzw. dem Pumpengehäuse bereitgestellte Volumenraum ist vom übrigen Spülraum 4 durch ein Flächensieb 14 getrennt. Mittels diesem Flächensieb 14 können gröbere Verschmutzungen zurückgehalten werden. Dabei ist es bevorzugt, das Flächensieb 14 mit einem Reservoir 14a auszurüsten, in welchem vom Flächensieb 14 zurückgehaltene Partikel, Verschmutzungen und/oder dergleichen Fremdstoffe während der Durchführung eines Spülprogramms gesammelt werden können.

Der Sammeltopf 12 ist mit einem Ablaufstutzen 13 ausgerüstet. Dieser ist an der tiefsten Stelle des Sammeltopfs 12 ausgebildet und dient nach erfolgreich durchlaufendem Spülprogramm der Wasser- bzw. Spülflottenentleerung. Dabei ist es bevorzugt, das Reservoir 14a des Flächensiebs 14 direkt oberhalb des Ablaufstutzens 13 auszugestalten, wie dies in Fig. 2 gezeigt ist. Im Reservoir 14a angesammelte Partikel, Verschmutzungen und/oder dergleichen Fremdstoffe können so bei einer Restwasserentleerung direkt über den Ablaufstutzen 13 abgepumpt werden.

Die Umwälzpumpe 15 verfügt über einen außerhalb des Sammeltopfes 12 ausgebildeten Motor 16. Dieser treibt über eine Pumpenwelle 18 das im Sammeltopf 12 angeordnete Pumpenrad 17 an. Dieses wirkt mit einem fest-, d.h. stillstehenden Leitrad 21 zusammen. Die im bestimmungsgemäßen Betriebsfall am Umfang des Pumpenrades 17 austretende Wassermenge wird durch das Leitrad 21 aufgefangen und in Richtung der Drehachse 9 umgelenkt und mit Bezug auf die Zeichnungsebene nach Fig. 2 nach oben in das Versorgungsrohr 24 eingeleitet, von wo aus dann eine Bedienung der Sprüharme 7, 8 und 28 stattfindet.

Im Sammeltopf 12 bzw. im Pumpengehäuse der Umwälzpumpe 15 können ferner Elektro- und/oder Dampfheizkörper in Form einer Heizeinrichtung 22 vorgesehen sein. Damit dient der Sammeltopf 12 nicht nur der Funktion als Ansaugvolumen für die Umwälzpumpe 15, er wird auch für die Unterbringung einer entsprechenden Heizeinrichtung 22 genutzt.

Gemäß einer bevorzugten Ausführungsform findet ein elektromotorischer Antrieb der Sprüharme 7, 8 und 28 der Sprüheinrichtung 23 statt. Zu diesem Zweck ist ein Motor 19 vorgesehen, der über eine Antriebswelle 20 an die verdrehbar gelagerten Sprüharm 7, 8 und 28 angeschlossen ist. Dabei ist es bevorzugt, dass die Verbindung der einzelnen Abschnitte 25, 26 und 27 des Versorgungsrohrs 24 kraftübertragend ausgebildet ist, so dass über einen Antrieb allein des unteren, zweiten Abschnitts 27 des Versorgungsrohrs 24 ein Antriebs sämtlicher Sprüharme 7, 8 und 28 erreicht ist.

Die Antriebswelle 20 erstreckt sich bevorzugter Weise entlang der Drehachse 9, zu welchem Zweck die Pumpenwelle 18 als Hohlwelle ausgebildet ist, durch die hindurch die Antriebswelle 20 zum Antrieb der Sprüharme 7, 8 und 28 geführt ist.

Gemäß einer alternativen Ausgestaltung der Erfindung kann ein Antrieb der Sprüharme 7, 8 und 28 auch von oben, d.h. deckenseitig erfolgen. Gemäß dieser Alternative sind ein Motor 31 und eine Welle 32 vorgesehen, die auf den oberen, d.h. ersten Abschnitt 25 des Versorgungsrohrs 24 und den damit gekoppelten Sprüharm 7 einwirken. Der Motor 19 und die Antriebswelle 20 können im Fall dieser alternativen Ausgestaltungsform entfallen.

Die Sprüheinrichtung 23 dient nicht nur der Beschickung von Spülgut mit Spülflotte, sondern auch der Beschickung des Spülguts mit Trocknungsluft. Zu diesem Zweck ist ein Lufteinlass 30 vorgesehen, der an ein in den Figuren nicht näher dargestelltes Gebläse angeschlossen ist. Über den Lufteinlass 30 kann vom Gebläse geförderte Luft in das Versorgungsrohr 24 geleitet werden, von wo aus dann eine Verteilung auf die einzelnen Sprüharme 7, 8 und 28 stattfindet.

Neben den in den Figuren gezeigten Sprüharmen 7, 8 und 28 kann die Sprüheinrichtung 23 auch andere Fluidabgabeeinrichtungen aufweisen, z.B. in Form von Anschlussstutzen, an die in einfacher Weise beispielsweise Schläuche durch Aufstecken angeschlossen werden können.

Die Besonderheit der erfindungsgemäßen Konstruktion liegt darin, dass das Verteilrohr 24 als zentrales Verteilrohr zur Beschickung der Sprüheinrichtung 23 mit Spülflotte oder Trocknungsluft dient, wobei das Versorgungsrohr 24 innerhalb des Spülraums 4 angeordnet ist. Dabei fallen die Drehachse 9 des Pumpenrades 17 der Umwälzpumpe 15 und die Drehachse 9 der Sprüharme 7, 8 und 28 zusammen und es ist vorgesehen, dass der von der Umwälzpumpe 15 geförderte Volumenstrom direkt über das mit der Umwälzpumpe 15 gekoppelte Versorgungsrohr 24 in die Sprüheinrichtung 23, d.h. die zugehörigen Sprüharme 7, 8 und 28 eingeleitet wird.

### Bezugszeichenliste

- 1: Spülautomat
- 2: Gehäuse
- 3: Spülbehälter
- 4: Spülraum
- 5: Beschickungsöffnung
- 6: Spülraumtür
- 7: Sprüharm
- 8: Sprüharm
- 9: Drehachse
- 10: Spülkorb
- 11: Gestell
- 12: Sammeltopf
- 13: Ablaufstutzen
- 14: Flächensieb
- 15: Umwälzpumpe
- 16: Motor
- 17: Pumpenrad
- 18: Pumpenwelle
- 19: Motor
- 20: Antriebswelle
- 21: Leitrad
- 22: Heizeinrichtung
- 23: Sprüheinrichtung
- 24: Versorgungsrohr
- 25: erster Abschnitt
- 26: dritter Abschnitt
- 27: zweiter Abschnitt
- 28: Sprüharm
- 29: Kupplungsstelle
- 30: Luftanschluss
- 31: Motor
- 32: Welle

## Patentansprüche

1. Reinigungs- und Desinfektionsautomat insbesondere für medizinische Instrumente und Geräte, mit einem einen Spülraum (4) bereitstellenden Spülbehälter (3) und einer im Spülraum (4) angeordneten Sprüheinrichtung (23) zur Beschickung von Spülgut mit Spülflotte, wobei die Sprüheinrichtung (23) zur Versorgung mit Spülflotte an ein Versorgungsrohr (24) angeschlossen ist, welches im Spülraum (4) verlaufend ausgebildet und einendseitig an eine Umwälzpumpe (15) angeschlossen ist,
**dadurch gekennzeichnet,**
**dass** das Versorgungsrohr (24) anderendseitig an ein Gebläse angeschlossen ist.

2. Automat nach Anspruch 1,
**dadurch gekennzeichnet,**
**dass** die Sprüheinrichtung (23) eine Mehrzahl von um eine gemeinsame Drehachse (9) verdrehbar gelagerten Sprüharmen (7, 8, 28) aufweist.

3. Automat nach einem der vorhergehenden Ansprüche,
**dadurch gekennzeichnet,**
**dass** sich das Versorgungsrohr (24) innerhalb des Spülraums (4) senkrecht entlang der Mittelachse des Spülraums (4) erstreckt.

4. Automat nach einem der vorhergehenden Ansprüche 1 bis 3,
**dadurch gekennzeichnet,**
**dass** sich das Versorgungsrohr (24) entlang der Drehachse (9) der Sprüharme (7, 8, 28) erstreckt.

5. Automat nach Anspruch 3 und 4,
**dadurch gekennzeichnet,**
**dass** die Mittelachse des Spülraums (4) und die Drehachse (9) der Sprüharme (7, 8, 28) zusammenfallen.

6. Automat nach einem der vorhergehenden Ansprüche,
**dadurch gekennzeichnet,**
**dass** das Versorgungsrohr (24) in fluiddicht miteinander verbindbare Abschnitte (25, 26, 27) unterteilt ausgebildet ist.

7. Automat nach Anspruch 6,
**dadurch gekennzeichnet,**
**dass** das Versorgungsrohr (24) einen ersten und einen zweiten Abschnitt (25, 27) aufweist, die boden- bzw. deckenseitig des Spülraums (4) angeordnet sind und jeweils einen Sprüharm (7, 8) bereit stellen.

8. Automat nach Anspruch 6 oder 7,
**dadurch gekennzeichnet,**
**dass** das Versorgungsrohr (24) einen zwischen erstem und zweitem Abschnitt (25, 27) angeordneten dritten Abschnitt (26) aufweist, der als Teil eines der Spülgutaufnahme dienenden und aus dem Spülraum (4) herausziehbaren Spülkorbs (10) ausgebildet ist.

## Claims

1. Automatic cleaning and disinfection machine, in particular for medical instruments and appliances, comprising a washing container (3) which provides a rinsing chamber (4), and a spray device (23) arranged in the rinsing chamber (4) for loading washware with washing solution, the spray device (23) being connected to a supply pipe (24) for supplying washing solution, which supply pipe is designed to extend in the rinsing chamber (4) and is connected, at one end, to a circulating pump (15), **characterised in that**
the other end of the supply pipe (24) is connected to a fan.

2. Automatic machine according to claim 1,
**characterised in that**
the spray device (23) comprises a plurality of spray arms (7, 8, 28) that are mounted such that they can rotate about a common axis of rotation (9).

3. Automatic machine according to either of the preceding claims,
**characterised in that**
the supply pipe (24) extends perpendicularly inside the rinsing chamber (4) along the central axis of the rinsing chamber (4).

4. Automatic machine according to any of the preceding claims 1 to 3, **characterised in that**
the supply pipe (24) extends along the axis of rotation (9) of the spray arms (7, 8, 28).

5. Automatic machine according to claims 3 and 4,
**characterised in that**
the central axis of the rinsing chamber (4) and the axis of rotation (9) of the spray arms (7, 8, 28) coincide.

6. Automatic machine according to any of the preceding claims,
**characterised in that**
the supply pipe (24) is divided into portions (25, 26, 27) that can be interconnected in a fluid-tight manner.

7. Automatic machine according to claim 6,
**characterised in that**
the supply pipe (24) comprises a first and a second portion (25, 27) which are arranged on the base and roof, respectively, of the rinsing chamber (4) and each provide one washing arm (7, 8) respectively.

8. Automatic machine according to either claim 6 or claim 7,
**characterised in that**
the supply pipe (24) comprises a third portion (26) arranged between the first and second portions (25, 27) which is formed as a part of a dishwasher rack (10) that functions as the washware receptacle and is designed to be able to be pulled out of the rinsing chamber (4).

## Revendications

1. Appareil automatique de nettoyage et de désinfection, en particulier pour instruments et appareils médicaux, avec une cuve de lavage (3) fournissant un espace de lavage (4) et avec un dispositif de pulvérisation (23) disposé dans l'espace de lavage (4) pour l'alimentation des articles à laver en eau de rinçage, dans lequel le dispositif de pulvérisation (23), pour l'alimentation en eau de rinçage, est raccordé à un tube d'alimentation (24) qui est constitué dans l'espace de lavage (4) et relié, à une extrémité, à une pompe de circulation (15),
**caractérisé en ce que**
le tube d'alimentation (24) est, à l'autre extrémité, raccordé à un ventilateur.

2. Appareil automatique selon la revendication 1,
**caractérisé en ce que**
le dispositif de pulvérisation (23) présente une pluralité de bras de pulvérisation (7, 8, 28) supportés de façon à pouvoir tourner autour d'un axe de rotation (9) commun.

3. Appareil automatique selon l'une des revendications précédentes,
**caractérisé en ce que**
le tube d'alimentation (24) s'étend à l'intérieur de l'espace de lavage (4) perpendiculairement le long de l'axe central de l'espace de lavage (4).

4. Appareil automatique selon l'une des revendications précédentes 1 à 3,
**caractérisé en ce que** le tube d'alimentation (24) s'étend le long de l'axe de rotation (9) des bras de pulvérisation (7, 8, 28).

5. Appareil automatique selon les revendications 3 et 4,
**caractérisé en ce que** l'axe central de l'espace de lavage (4) et l'axe de rotation (9) des bras de pulvérisation (7, 8, 28) coïncident.

6. Appareil automatique selon l'une des revendications précédentes,
**caractérisé en ce que**
le tube d'alimentation (24) est constitué de façon divisée en tronçons (25, 26, 27) pouvant être raccordés les uns aux autres de façon étanche aux fluides.

7. Appareil automatique selon la revendication 6,
**caractérisé en ce que**
le tube d'alimentation (24) présente un premier et un deuxième tronçon (25, 27) qui sont disposés côté fond et respectivement côté sommet de l'espace de lavage (4) et fournissent respectivement un bras de pulvérisation (7, 8).

8. Appareil automatique selon la revendication 6 ou 7,
**caractérisé en ce que**
le tube d'alimentation (24) présente un troisième tronçon (26), disposé entre le premier et le deuxième tronçon (25, 27), qui est constitué en tant que partie d'un panier de lavage (10) servant à recevoir les articles à laver et pouvant être extrait de l'espace de lavage (4).
